# EUROPEAN PATENT APPLICATION

(11) **EP 2 439 668 A1**
(43) Date of publication of application: **11.04.2012**
(21) Application number: 11184379.3
(22) Date of filing: 07.10.2011
(51) Int. Cl.: G06F 19/00

(54) **Computer-implemented system and method for mediating patient-initiated physiological monitoring**

(30) Priority: 08.10.2010 US 901455
(71) Applicant: Cardiac Science Corporation, Bothell, WA 98021 (US)
(72) Inventor: Bardy, Gust H., Carnation, WA Washington 98014 (US); Bishay, Jon Mikalson, Seattle, WA Washington 98119 (US)
(74) Representative: Curley, Donnacha John

(57) **Abstract**

Access to key diagnostic medical devices (33) requires pre-approval by a screening physician. A computer-implemented system and method for mediating patient-initiated physiological monitoring and ensuring appropriate follow up, bypassing the need for a screening physician, are provided. A request for a medical monitoring device (33) from a patient (31) at a point of prescriptive medicine dispensing is entered. The patient (31) is enrolled in a medical service network (19) that includes healthcare providers and medical services, and one or more on-call prescribing physicians (22). The patient (31) is interfaced in real time with one of the prescribing physicians (22). Upon physician approval, the monitoring device (33) is dispensed to the patient (31) at the point of dispensing. Following monitoring, data from the monitoring device (33) is retrieved for the medical service network (19). The retrieved data is evaluated for medical concerns. Based on findings (52) from the evaluation made, the patient (31) is referred for medical care through the healthcare providers and medical services.

## Description

### Field

This application relates in general to medical device monitoring and, in particular, to a computer-implemented system and method for mediating patient-initiated physiological monitoring.

### Background

Ambulatory monitoring is used to continuously monitor and record physiological data while a patient engages in activities of daily living. Extended monitoring also collects physiological data as the patient goes about his life, but can be discontinuous with gaps occurring in the recorded data. Both forms of monitoring are typically used outside of a clinical environment to capture the effect of real world stimuli on a patient's condition. Both ambulatory and extended monitoring reflect a statistically stronger likelihood of capturing sporadic symptoms than would be possible by monitoring a patient in-clinic alone. Long term monitoring helps with temporally correlating physiological changes to patient complaints, course of medical treatment, and drug therapeutic efficacy. As well, the physiological data recorded through such monitoring can be used in diagnosing and treating chronic disease, medical conditions with non-regularly occurring symptoms, and disorders with acute but non-sustained onset.

Use of ambulatory and extended monitoring of patient physiology is used more often in areas of specialized medicine focused on diagnosing and treating asymptomatic disorders than in primary care. In cardiology, for instance, ambulatory electrocardiographic (ECG) monitors are used in diagnosing arrhythmias. Sleep disorder clinics use extended polysomnographic monitors, which are worn only at night, to confirm or refute the presence of sleep apnea. In endocrinology, blood glucose monitors, some employing subcutaneous sensors, are used to monitor treatment of diabetes, which is reliant on the quality of self-care and the patient's physical well-being, diet, activity level, and insulin dosing.

Depending upon type, ambulatory and extended monitors are available either over-the-counter or by a physician's prescription only. Under managed health care, a patient must generally first be evaluated by a primary care physician before a prescription is written. These physicians typically first undertake their own diagnostic testing and screening and may conclude that monitoring is unnecessary. As a result, a patient who has experienced sporadic symptoms may be barred by his primary care physician from undergoing ambulatory or extended monitoring, albeit not necessarily by nefarious intent. The patient must either persist in convincing his primary care provider to permit monitoring, or shoulder the costs of outside-the-network medical care in pursuit of monitoring without primary care provider referral and at the risk of non-reimbursement.

U.S. Patent application, Publication No. 2007/0255153, filed November 1, 2007, to Kumar et al.; U.S. Patent application, Publication No. 2007/0225611, filed February 6, 2007, to Kumar et al.; and U.S. Patent application, Publication No. 2007/0249946, filed February 6, 2007, to Kumar et al. disclose a non-invasive cardiac monitor and methods of using continuously recorded cardiac data. A heart monitor suitable for use in primary care includes a self-contained and sealed housing. Continuously recorded cardiac monitoring is provided through a sequence of simple detect-store-offload operations that are performed by a state machine. The housing is adapted to remain affixed to a mammal from at least seven days up through 30 days. The heart monitor can include an activation or event notation button, the actuation of which increases the fidelity of the ECG information stored in the memory. The stored information can be retrieved and analyzed offline to identify ECG events, including determining the presence of an arrhythmia.

Finally, U.S. Patent application, Publication No. 2008/0284599, filed April 28, 2006, to Zdeblick et al. and U.S. Patent application, Publication No. 2008/0306359, filed December 11, 2008, to Zdeblick et al., disclose a pharma-informatics system for detecting the actual physical delivery of a pharmaceutical agent into a body. An integrated circuit is surrounded by pharmacologically active or inert materials to form a pill, which dissolve in the stomach through a combination of mechanical action and stomach fluids. As the pill dissolves, areas of the integrated circuit become exposed and power is supplied to the circuit, which begins to operate and transmit a signal that may indicate the type, A signal detection receiver can be positioned as an external device worn outside the body with one or more electrodes attached to the skin at different locations. The receiver can include the capability to provide both pharmaceutical ingestion reporting and psychological sensing in a form that can be transmitted to a remote location, such as a clinician or central monitoring agency.

Therefore, a need remains for a way to provide ambulatory or extended monitoring to patients with perceived needs that is not reliant on primary care provider discretion and willingness to prescribe, yet that is viable within managed care restrictions.

### Summary

A computer-implemented system and method for enabling a patient to initiate self-medical physiological monitoring is provided. An online medical service network electronically interfaces to various points-of-sale. One or more prescribing physicians or health care professionals, such as registered nurses, may be retained by the medical service network. When a patient requests purchase or dispensing of a medical monitoring device, such as an ambulatory monitor or a monitor for extended use, an operator at the point of sale, for instance, a pharmacist, enrolls the patient into the medical service network. When a prescription or medical consultation is necessary or recommended, the pharmacist places the patient into contact with one of the prescribing physicians. Following an interview that occurs in real time while the patient is still at the point of sale, the prescribing physician writes a prescription, if appropriate, or provides a recommendation. The patient then receives the requested monitor for self-use. Thereafter, physiological data recorded by the monitor is retrieved by a server operated by the medical service network and evaluated. Upon findings indicating a need for follow up, the patient is automatically notified of an appointment with a medical specialist or provided medical services to address the medical need identified without first having to see his primary care physician to obtain a referral or permission to receive the follow up care.

One embodiment provides a computer-implemented system and method for mediating patient-initiated physiological monitoring. A patient is enrolled in an online medical service network that includes healthcare providers and medical services. A request for a physiological monitoring device is received from the patient. The monitoring device is directly dispensed to the patient, after which the patient undertakes self-monitoring through use of the medical monitoring device. Electronically-recorded physiological data is retrieved from the monitoring device following completion of the self-monitoring by the patient. The retrieved physiological data is evaluated against diagnostic criteria for medical concerns that include health disorders with detectable symptoms. Based on findings from the evaluation made, the patient is referred for follow up care through the healthcare providers and medical services.

A further embodiment provides a computer-implemented system and method for mediating patient-initiated physiological prescriptive monitoring. A patient is enrolled in a medical service network comprising healthcare providers and medical services. The patient is designated for physiological monitoring using a monitoring device available only by a medical prescription. The patient is interfaced with a prescriptive authority at a point of sale. Upon issuance of the medical prescription by the prescriptive authority, the monitoring device is dispensed to the patient, after which the patient undertakes self-monitoring through use of the monitoring device. Electronically-recorded physiological data is retrieved from the monitoring device following completion of the self-monitoring by the patient. The retrieved physiological data are evaluated against diagnostic criteria for medical concerns that include of health disorders with detectable symptoms. Based on findings from the evaluation made, the patient is referred for follow up care through the healthcare providers and medical services.

A still further embodiment provides a computer-implemented system and method for mediating patient-initiated physiological non-prescriptive monitoring. A patient is enrolled in a medical service network that includes healthcare providers and medical services. The patient is designated for physiological monitoring using a monitoring device available without need of a medical prescription. The monitoring device is provided to the patient at a point of sale, after which the patient undertakes self-monitoring through use of the monitoring device. Electronically-recorded physiological data is retrieved from the monitoring device following completion of the self-monitoring by the patient. The retrieved physiological data is evaluated against diagnostic criteria for medical concerns that includes health disorders with detectable symptoms. Based on findings from the evaluation made, the patient is referred for follow up care through the healthcare providers and medical services.

Thus, patients are able to obtain a medical monitoring device under medical prescription, when required, or over-the-counter with responsible medical supervision and medical service network follow up thereafter. A primary care provider is not a required part of the process, yet the patient is still referred as appropriate to medical specialists or medical services as appropriate, if the monitoring device demonstrates a reason for referral. Patients save both the costs and inconvenience of undertaking intermediate diagnostic testing, as well as avoid the risk of non-reimbursement that arises when they seek assistance outside of their managed care plan. Moreover, patients can gain access to specialist care that is increasingly difficult to obtain.

Primary care physicians are empowered with a type of ambulatory ECG monitoring that, in conjunction with a referral center, ensures proper data interpretation and medical follow up. A primary care physician need only apply an ambulatory ECG monitor in-clinic or provide a monitor to a patient through a prescription called into a pharmacy or other dispensary point of sale. Subspecialty expertise in arrhythmia diagnosis need not be resident in the provider's clinic, nor must the patient be referred to a separate ambulatory ECG testing laboratory. The low cost of each monitor encourages use when patient symptoms urge access to ambulatory ECG monitoring data. The backup system of support for the general physician helps minimize the risk of misdiagnosis and the need to even establish a referral, which is often not a simple decision or a simple process to ensure. Additionally, the combination of low cost and convenience of access to expertise encourages testing when appropriate to evaluating new medications or other changes important for the conduct of high-quality medical care.

Another key feature is that patients are empowered with the ability to self-screen a potential arrhythmic condition through ambulatory ECG monitoring. Access to cardiac rhythm expertise is difficult for a variety of reasons. Patients save both the costs and inconvenience of undertaking intermediate diagnostic testing, as typically required when undergoing conventional Holter-type ambulatory ECG monitoring, as well as avoid the risk of non-reimbursement that arises when they seek help outside their managed care plan. Patients are able to stay informed of their test results and follow on care without having to passively wait for follow up to occur. Moreover, wasted time is avoided by all interested parties.

Finally, as part of the system employed by primary care providers, cardiac specialists are empowered with receiving complete patient referrals and critical ECG data that enable them to effectively diagnose and treat arrhythmic conditions without the usual repetitive phone calls and requests to access medical information between doctors offices. Medical information and patient-generated diary entries are communicated to the referral center as part of the ambulatory ECG monitoring process, which is provided to cardiac specialists as part of a complete referral.

Still other embodiments will become readily apparent to those skilled in the art from the following detailed description, wherein are described embodiments by way of illustrating the best mode contemplated. As will be realized, other and different embodiments are possible and the embodiments' several details are capable of modifications in various obvious respects, all without departing from their spirit and the scope. Accordingly, the drawings and detailed description are to be regarded as illustrative in nature and not as restrictive.

### Brief Description of the Drawings

FIGURE 1 is a diagram showing, by way of example, a system for mediating patient-initiated physiological monitoring in accordance with one embodiment.
FIGURE 2 is a diagram showing, by way of example, point of sale medical monitoring device dispensation in accordance with one embodiment.
FIGURE 3 is a diagram showing, by way of example, self-service medical monitoring device dispensation in accordance with a further embodiment.
FIGURE 4 is a diagram showing, by way of example, medical monitoring device data retrieval, processing, and follow up.

### Detailed Description

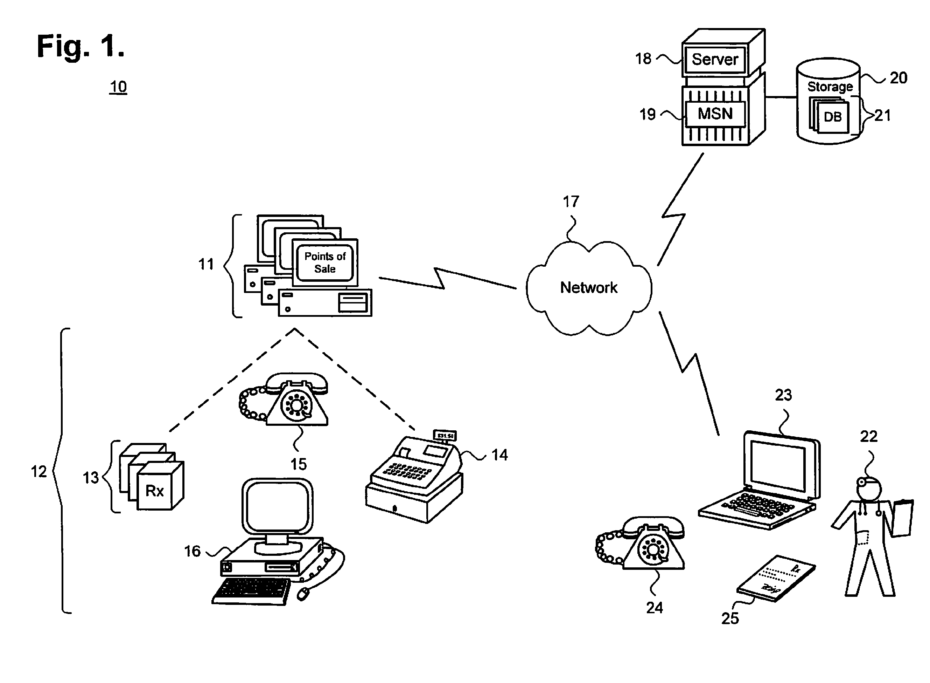

Patients are afforded direct access to ambulatory and extended monitoring devices, particularly those types of devices not available over-the-counter and which require a physician's prescription, through an online medical service network. Monitoring and diagnosis thus becomes fully patient-initiated. FIGURE 1 is a diagram showing, by way of example, a system for mediating patient-initiated physiological monitoring 10 in accordance with one embodiment. The system 10 operates on computing devices that include servers and clients which may be computer workstations, personal computers, personal digital assistants, programmable or "smart" mobile telephones, such as an iPhone or Blackberry, respectively licensed by Apple Inc., Cupertino, CA and Research In Motion, Waterloo, Ontario, Canada, intelligent digital media players, mobile tablet computers, or other programmable stationary or portable electronic devices. Each computing device includes those components conventionally found in general purpose programmable devices, such as a central processing unit, volatile memory, input and output ports, user display, keyboard or other input device, network interface, and non-volatile mass storage. Other components are possible.

Ambulatory and extended medical monitoring devices 13 are available to patients through commercial points of sale 11, including traditional points-of-sale for medical goods, such as pharmacies; non-traditional points-of-sale, such as consumer electronics stores; self-service outlets (dispensing apparatus), such as vending machines and kiosks; and other forms of retail marketing and goods outlets or distribution points, including online sales. Each point of sale 11 includes several components 12 that perform equivalent functions, but are implemented based on the nature of the point of sale. For instance, a traditional "brick-and-mortar" store manned by sales staff typically includes a physical cashiering station with a cash register 14 or point of sale terminal, whereas an online point of sale would ordinarily have a secure Web page (not shown) through which to complete online purchases. Each point of sale 11 also includes a telephone 15 or other type of real time communications means, which could also be provided by the patient through a personal mobile telephone and the like. Each point of sale 12 additionally includes a computer workstation 16 that is interfaced over a network 17 to a centralized server 18, which is operated by a medical service network ("MSN") 19 that includes healthcare providers and medical services. Similarly, the patient might provide their own computing device, for instance, by using a Web-enabled smart telephone or tablet computer. The network 17 can be either a dedicated private data communications circuit or publicly-available data communications network, such as the Internet, and can include wired, wireless, or combined forms of data transmission medium.

The medical service network 19 is a centralized online service that interfaces patients with physicians, registered nurses, and other providers of healthcare and medical services, such as described in commonly-assigned U.S. Patent application, entitled "Computer-Implemented System and Method for Facilitating Patient Advocacy through Online Healthcare Provisioning," Serial No. 12/901,433, filed October 8, 2010, pending, the disclosure of which is incorporated by reference. The medical service network 19 maintains a database 21 of general medicine physicians, specialized medicine physicians, diagnostic criteria, and authorized points of sale that is electronically maintained in storage 20 coupled to the server 18. As well, relationships between the general medicine physicians and specialized medicine physicians are formed into a patient referral tree stored in records in the database 21.

In general, medical monitoring devices refer to a class of medical equipment for electronically recording physiological data that can be useful in the diagnosis and care of health disorders with detectable symptoms. These devices can include electrodes, sensors, and input ports that are either built directly into the monitor, or which are connected through leads or wireless means, through which physiological data, as well as other information, can either be sensed, received, or manually input. As well, a single monitoring device may be collaboratively interfaced with other monitoring devices, subcomponents, such as sensors, or even therapeutic medical devices, such as insulin pumps and implantable pacemakers and defibrillators.

Some types of medical monitoring devices are classified by the U.S. Food & Drug Administration (FDA) as Class 2 medical devices that are subject to special controls that include labeling requirements, mandatory performance requirements, and post market surveillance. Availability and use are controlled. When mandated, a medical prescription issued by a prescriptive authority, generally a licensed physician, may be required to obtain a monitoring device 13. Depending upon applicable law, the prescriptive authority may be a physician or other authorized individual with whom the patient has consulted prior to receiving a prescription, but need not necessarily be the same physician with whom the patient has an in-clinic relationship. For instance, the prescriptive authority could be a physician available over the telephone or by online communications with whom the patient connects by virtue of a medical service network. In some instances, the prescribing physician may be in contact with the patient's customary care provider, typically his primary care or specialist physician, either during or following issuance of the prescription.

Other types of medical monitoring devices are not subject to prescriptive control. These types of monitoring devices may be of a less invasive or medically-difficult nature. For instance, a Holter-type ambulatory electrocardiographic monitor has numerous electrodes connected to a recording unit through wired leads. Placement of the leads is critical to recording a usable electrocardiogram and the average lay patient normally lacks the skills to properly place the leads and thereby ensure quality electrocardiographic recording. Consequently, Holter-type monitors are ordinarily limited to prescriptive use. Other monitoring devices, such as glucose and heart rate monitors, are more robust to and forgiving of lay use. Placement of a blood specimen or blood pressure cuff is less critical to achieving useful monitoring results than electrocardiograph leads. As a result, these devices often may be purchased over-the-counter through retailers of medical supplies and other goods.

Where prescriptive dispensation is required, one or more prescriptive authorities, that is, prescribing physicians 22, are also retained by the medical service network 19 to assist points of sale in dispensing monitoring devices 13 to patients directly. Each prescribing physician 22 is interfaced over the network 17 through a computer workstation 23 to the centralized server 18. Each prescribing physician 22 also has a telephone 24 or other type of real time communications means on hand and authority to issue medical prescriptions 25.

Conventionally, under managed care, primary care providers, who are normally internal medicine and family practice physicians, first undertake their own diagnostic testing and screening before referring a patient to a medical specialist for further testing. They must additionally be willing to advocate on behalf of the patient's condition in favor of more in-depth testing and care. In light of the backend burden thus imposed, primary care physicians may understandably decline to make a medical specialist referral, particularly where no outward symptoms are present in-clinic and the patient's condition may be assignable to benign causes, even though an occult and more serious disorder may be behind the patient's complaint. Evaluating the patient thoroughly often involves the ordering of tests, like ambulatory ECG monitoring, that are easy to avoid instead of undertaking the clinical appointments, paperwork, and follow up burdens that such testing generally entails.

Conversely, when a primary care physician decides to refer, the patient must work through his primary care provider to have an ambulatory or extended monitor ordered or prescribed; have the monitor placed, read and removed; have the recorded physiological data interpreted; and undertake follow up on any diagnostic findings made, either with his own primary care provider, with a specialist physician, or both. All of these tasks require money, time and, involvement of several distinct entities, including the primary care provider, the laboratory that dispensed the monitoring device, and the medical specialist charged with diagnostic over-read and follow up. At a minimum, multiple communications and exchanges of information are required, permissions secured, and disclosures made before the patient actually has his concern addressed. Undertaking these various steps can frustrate the patient by creating unnecessary cost, friction, anxiety, uncertainty, and waiting.

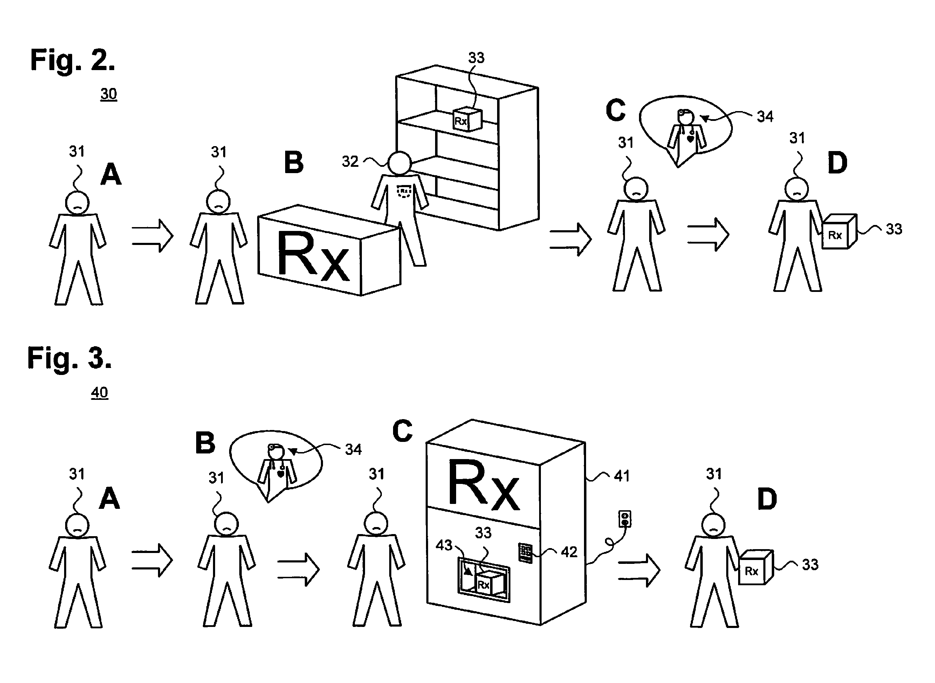

With the assistance of the medical service network, a patient can receive a medical monitoring device directly at a point of sale without having to first obtain a prescription or tacit permission from his primary care provider, or undergoing other managed care mandated coordination, such as a referral to a monitoring laboratory. FIGURE 2 is a diagram showing, by way of example, point of sale medical monitoring device dispensation 30 in accordance with one embodiment. Initially, a patient 31 realizes that he has symptoms possibly caused by a medical disorder of some type (step A). Alternatively, the patient 31 may simply feel a need to have some form of monitoring performed. Prior to deciding to undertake self-care physiological monitoring, the patient may opt to utilize "on-call" patient services (not shown), where the patient can talk to physicians, nurse practitioners, or other health care professionals about the health concerns, such as described in commonly-assigned US patent application, entitled "Computer-Implemented System and Method for Facilitating Patient Advocacy through Online Health Care Provisioning," cited *supra.* The discussion may potentially occur by telephone, or be conducted electronically, including by text messaging or email.

The monitoring can be related to various types of health disorders or conditions. For instance, for diagnosing a potential heart rhythm disorder, the patient 31 may want to use an ambulatory ECG monitor, such as described in commonly-assigned U.S. Patent application, entitled "Ambulatory Electrocardiographic Monitor and Method of Use," Serial No. 12/901,444, filed October 8, 2010, pending, and commonly-assigned U.S. Patent application, entitled "Ambulatory Electrocardiographic Monitor for Providing Ease of Use in Women and Method of Use," Serial No. 12/901,428, filed October 8, 2010, pending, the disclosures of which are incorporated by reference. In contrast, the patient 31 may want to simply check her blood glucose, heart rate, or determine whether she may be pregnant. Other forms of monitoring are possible.

Here, the patient 31 physically proceeds to a point of sale, typically a pharmacy or other retail outlet, to purchase or obtain an ambulatory or extended monitoring device. Upon request by the patient 31 for a monitoring device 33 (step **B**), staff at the point of sale 12, typically, a pharmacist 32, contacts the medical service network 19 (shown in FIGURE 1) through the computer workstation 16 and enrolls the patient 31 into the medical service network 19, if not already enrolled. In a further embodiment, the point of sale includes a patient information center, such as a merchandise display, kiosk, or other form of information provisioning outlet. The patient information center provides educational information to the patient 31 that helps him to understand whether undertaking monitoring may be appropriate. The information can be through the form of questionnaires or other screening materials with which the patient 31 can interact, either electronically using a suitable input means such as a keypad or manually. For instance, a question-and-answer format presented on a display, such as commonly used with Automated Teller Machines or ATMs, could be used to query the patient and help determine a course of action. In this way, the input means of the dispensing apparatus may facilitate self-initiated diagnosis by the patient through completion of the screening materials by appropriate responses inputted on the input means prior to the dispensing of the self-monitoring device.

The patient information center, in conjunction with the pharmacist 32 and medical service center staff can help the patient decide upon and obtain monitoring equipment. Additionally, if the monitoring device 33 requires a prescription, the patient 31 is put into contact with a specialist physician 34 (step **C**), who is authorized to prescribe such monitoring devices. Ordinarily, the specialist physician 34 is not aligned with the patient's primary care physician and works independently through the medical service network. Patient-to-prescribing physician contact can be over their respective telephones 15, 24 or other type of real time communications, such as the patient's mobile telephone. The prescribing physician 34 interviews the patient 31 and determines the medical propriety of prescribing or authorizing use of the monitoring device 33 to the patient 31. If medically appropriate, the prescribing physician 34 electronically issues a prescription 25 to the patient 31 through his computer workstation 23 to be filled at the point of sale 12 by the pharmacist 32. If a prescription is not required, the prescribing physician 34 can provide advice to the patient 31, either over the telephone or through written guidance sent via his computer workstation 13 or originating from the server 18. The prescription 25 or written guidance is received by the point of sale computer workstation 16 and the ambulatory monitoring device 33 is dispensed to the patient 31 (step **D**), who then undertakes physiological monitoring.

Person-to-person interfacing may not always be necessary prior to undertaking physiological monitoring. FIGURE 3 is a diagram showing, by way of example, self-service medical monitoring device dispensation 40 in accordance with a further embodiment. For instance, if no prescription or other medical guidance is required, the patient 31 in need (step **A**) may simply purchase the monitoring device 33 directly from a vending machine 41 (step **C**) or equivalent form of self-service (dispensing apparatus) equipment or outlets that dispenses the monitoring device 33 directly to the patient 31 without requiring physical staffing or direct human interaction. The patient 31 may choose to first consult with a physician 34 (step **C**) before making his purchase. The vending machine 41 includes a set of input controls 42 to transact the purchase and, where required, authenticate the patient's identity and eligibility, such as where use of the monitoring device 33 is restricted to adults. The patient 31 will need to be enrolled with the medical service network 19 at some point to ensure that proper evaluation of the physiological data recorded and medical follow up occurs as appropriate following completion of monitoring (step D). The vending machine-style of monitoring device dispensing could also be used where a prescription is required, for instance, by requiring the prescribing physician 34 to provide a security code to the patient 31 that must be successfully entered through the vending machine's controls 42 as a precondition to dispensing 43 of the monitoring device 33.

In a further embodiment, medical information from a patient's electronic medical records can be provided with the monitoring device 33 for use in post-monitoring evaluation and patient care. The medical information can be electronically encoded and stored directly into the recording circuitry of the monitoring device 33 or on a memory circuit provided with the monitoring device 33, yet maintained as a separate memory space from the memory spaced used to store recorded electrocardiograms. Other ways of physically combining together the electronically-stored patient medical information and the monitoring device 33 are possible.

The medical information could also be provided in a storage medium that is physically separate from the monitoring device 33, but delivered at the same time by the patient as part of the monitoring device 33 following monitoring. The storage medium and monitoring device 33 are preferably paired together to ensure that both are delivered. For instance, the patient medical information could be provided with the sealable envelope. The encoding of the patient's electronic medical records can be applied to a memory device embedded into the sealable envelop, or could be physically applied to or included with the sealable envelope using a printed medium. Finally, the select medical information can be separately relayed, such as through email, via a remote server, or by separate physical mailing, and matched up at the point of evaluation at the medical service network upon receipt of the monitoring device 33 following monitoring. Other forms of data encoding and relay could be used.

In a still further embodiment, the patient 31 is provided with a diary in which to chronicle has subjective observation contemporaneous to monitoring, such as described in commonly-assigned U.S. Patent application, entitled "Computer-Implemented System and Method for Evaluating Ambulatory Electrocardiographic Ambulatory Monitoring of Cardiac Rhythm Disorders," Serial No. 12/901,461, filed October 8, 2010, pending, the disclosure of which is incorporated by reference. The patient 31 is either provided with a form of electronic or traditional paper diary, or instructions on accessing a dictation service that records the diary entries for the patient. The diary entries help temporally link objective symptomatic data recorded by the monitoring device to subjective observations made by the patient concerning his activity and physical complaints during the monitoring period. Data entries are particularly helpful in placing patient's symptomatic complaints in context, as clinic visits may be separated by several months and recollection of circumstances attendant to complaints may be overlooked or forgotten, unless written down as the diary entries around the time of occurrence. The diary can be implemented in a form of software, technology-assisted dictation, or conventional writing that is later electronically transcribed. The diary entries can be temporally matched to any detectable symptoms present in physiology recorded by a monitoring device, which can assist with pairing physiological symptoms to the patient's activities of daily living and contemporaneous symptomatic complaints. Other uses of the diary entries and patient medical history are possible.

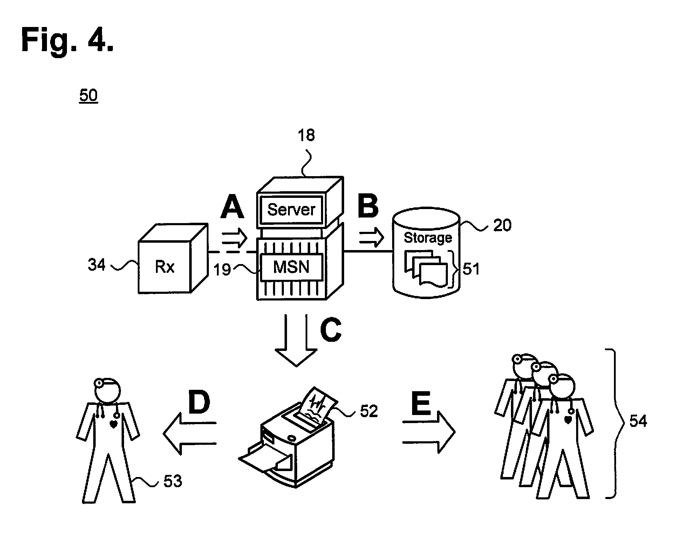

Upon completion of self-monitoring, the patient undertakes follow up. FIGURE 4 is a diagram showing, by way of example, medical monitoring device data retrieval, processing, and follow up 50. In contrast to conventional medical services under managed care, follow up is instead provided through the medical service network 18, which electronically receives physiological data recorded by the monitoring device 33 into the server 18 (step **A**), as well as an electronic facsimile of the patient's dairy entries, where applicable. The recorded physiological data 51 is archived into the storage 20 coupled to the server 18 (step **B**) and is evaluated by the server 18 for diagnostic findings 52 (step **C**), such as described in commonly-assigned U.S. Patent application, Serial No. 12/901,461, *Id.* If required, the recorded physiological data 54 and, if used, daily entries, are electronically provided to a consulting physician 53 (step **D**), who makes a formal medical diagnosis. The consulting physician 53 may also be provided with additional information concerning the patient 31, such as an electronically-stored medical history or other data.

One challenge persistently facing physicians is a lack of patient medical documentation, such as prior and current tests or laboratory results, medical history, and subjective patient's complaints. Such information can aid diagnosis, especially when they are available to a medical specialist to whom a patient has been referred. Providing the additional patient's medical information from the beginning of the referral streamlines the care giving process and avoids unnecessary follow-up appointments, when necessitated by a need to backfill already existing yet not immediately available patient's medical information. Finally, based on medical need, the patient 31 can be directly referred for follow up medical care to healthcare providers 54 (step **E**) in medical specialties corresponding to the nature of the ambulatory monitoring provided.

Follow up by the medical service network 19 can include selecting a suitable medical specialist where an affiliation with his general medicine physician does not exist, setting an appointment for the patient 31, and ensuring patient compliance with seeing the medical specialist. Other forms of medical service network-initiated follow up are possible. As a result, the patient is able to initiate medical monitoring of a potential medical disorder and receive specialized medical attention without having to see his primary care provider or work through the needless steps of managed care thus entailed. Additionally, the patient could undertake the use of a series of monitoring devices over the course of several months to establish a trending analysis, which can be particularly helpful in diagnosing sporadic ectopic symptoms or determine the efficacy of medications or other changes in patient care.

Under the managed care model of reimbursement, the primary care provider and medical specialist receive service fees paid by the managed care plan provider. Here, however, multiple entities are participating in a single patient care event and the other allocation of payment for services is distributed over a wide range of participants. The entities include the point of sale location, that is, the pharmacy or over-the-counter retailer, the medical service network, the medical specialist performing an over read of the physiological data collected by each monitoring device, the monitoring device manufacturer, and the data communication network provider, which facilitates the dialogue between the patient, point of sale, medical service network, and consulting physician. Additionally, service fees may be paid to "on-call" patient service providers with whom the patient discusses health concerned prior to undertaking self-care physiological monitoring. The "on-call" patient services may be provided as a patient service by the medical services network, or may be a separate entity. Where the monitoring device includes a wireless event monitor, the monitored data is wirelessly transmitted over the wireless carrier's data network and a service fee may be owed in compensation for the network access period. As well, the point of sale location dispenses monitoring devices, a third-party builds the monitoring devices, a patient services network interprets data recorded by monitoring devices, a telephone service provider, whether wireless or wired, communicates the monitoring data, and a data archival or storage provider, such as a server "cloud" farm, stores data, each entity of whom may be owed reimbursement for services rendered.

While the invention has been particularly shown and described as referenced to the embodiments thereof, those skilled in the art will understand that the foregoing and other changes in form and detail may be made therein without departing from the spirit and scope.

## Claims

1. A computer-implemented method (10) for mediating patient-initiated physiological monitoring, comprising:
enrolling a patient (31) in an online medical service network (19) comprised of healthcare providers and medical services;
receiving a request for a physiological monitoring device (33) from the patient (31) and directly dispensing the monitoring device (33) to the patient (31), after which the patient (31) undertakes self-monitoring through use of the medical monitoring device (33);
retrieving electronically-recorded physiological data (51) from the monitoring device (33) following completion of the self-monitoring by the patient (31);
evaluating the retrieved physiological data (51) against diagnostic criteria for medical concerns comprised of health disorders with detectable symptoms; and
based on findings (52) from the evaluation made, referring the patient (31) for follow up care through the healthcare providers and medical services.

2. A method according to Claim 1, further comprising:
contracting one or more on-call prescribing physicians (22) as part of the medical service network (19);
interfacing the patient (31) in real time with one of the prescribing physicians (22);
conditioning the dispensing of the monitoring device (33) upon issuance of the medical prescription by the prescribing physician.

3. A method according to Claim 1 or claim 2, wherein the act of dispensing comprises dispensing the monitoring device (33) through a vending machine or self service kiosk.

4. A method according to any preceding claim, further comprising at least one of:
retrieving an electronically-stored medical history for the patient (31) and including the medical history in the evaluation; and
retrieving an electronically-stored diary entered by the patient (31) contemporaneous to the self-monitoring and including the diary in the evaluation.

5. A method according to any preceding claim, further comprising referral to a specialist, wherein the referral comprises electronically providing the retrieved electronically-recorded physiological data to the specialist.

6. A method according to Claim 5, wherein
a) the monitoring device (33) comprises a heart monitor, the method further comprising identifying indications of heart rhythm disorders in the retrieved data and wherein the medical care referred comprises referral to a cardiologist or cardiac electrophysiologist,
b) the monitoring device (33) comprises a polysomnography monitor, the method further comprising identifying patterns of sleep disorder in the retrieved data, wherein the medical care referred comprises referral to a sleep disorder medical specialist or a neurologist,
c) the monitoring device (33) comprises a blood glucose monitor, the method further comprising identifying elevated levels of or irregular patterns of blood glucose in the retrieved data, wherein the medical care referred comprises referral to an endocrinologist or an internist,
d) the monitoring device (33) comprises a blood pressure monitor, the method further comprising identifying elevated or reduced patterns of blood pressure in the retrieved data, wherein the medical care referred comprises referral to an nephrologist, internist or cardiologist.

7. A system (10) for mediating patient-initiated physiological monitoring, the system comprising a server, a database and one or more clients and a dispensing apparatus: wherein the server is configured to:
a) receive enrollment data for a patient from a client and being adapted to store said enrollment data in the database;
b) receive an electronic request for a physiological monitoring device (33) from the patient (31), wherein the system is configured to issue a request to the dispensing apparatus to cause the direct dispensing of a monitoring device (33) to the patient (31), after which the patient (31) can undertake self-monitoring through use of the medical monitoring device (33);
c) receive retrieved electronically-recorded physiological data (51) from the monitoring device (33) following completion of the self-monitoring by the patient (31);
d) evaluate the retrieved physiological data (51) against diagnostic criteria for medical concerns comprised of health disorders with detectable symptoms; and based on findings (52) from the evaluation made,
e) referring the patient (31) for follow up care by a specialist by electronically transmitting the retrieved electronically-recorded physiological data to a client machine specialist.

8. A system according to claim 7, wherein the dispensing apparatus is a self service kiosk or vending machine.

9. A system according to claim 7 or claim 8, wherein the dispensing apparatus is configured to only dispense the monitoring device upon the provision of a code provided to them by a dispensing physician.

10. A system according to any one of Claims 7 to 9, wherein the system is further configured to match medical information previously stored on the database for the patient (31) to the retrieved physiological data (51) and to provide the medical information online to the healthcare providers and medical services.

11. A system according to any one of claims 7 to 10, method according to Claim 12, further comprising the monitoring device, wherein the monitoring device is configured to store medical information comprising one or more of monitored patient identification data, prior electrocardiograms, laboratory results, physical examination results, exercise testing results, and medical history.

12. A system according to any one of claims 7 to 11, further comprising an electronically stored diary for the patient wherein the diary allows the patient to record entries into the diary contemporaneous to the self-monitoring;
and wherein the system is configured to temporally match the diary entries to the retrieved physiological data (51) and to pair the detectable symptoms with the diary entries.

13. A system according to any one of claims 7 to 11, wherein the dispensing apparatus comprises a display and the system is configured to display screening materials regarding the health disorders to the patient on the display; and where the dispensing apparatus further comprising an input means for facilitating self-initiated diagnosis by the patient (31) through completion of the screening materials by appropriate responses inputted on the input means prior to the self-monitoring.

14. A computer-implemented method for mediating patient-initiated physiological prescriptive monitoring, comprising:
enrolling a patient (31) in a medical service network (19) comprising healthcare providers and medical services;
designating the patient (31) for physiological monitoring using a monitoring device (33) available only by a medical prescription;
interfacing the patient (31) with a prescriptive authority at a point of sale;
upon issuance of the medical prescription by the prescriptive authority, dispensing the monitoring device (33) to the patient (31), after which the patient (31) undertakes self-monitoring through use of the monitoring device (33);
retrieving electronically-recorded physiological data (51) from the monitoring device (33) following completion of the self-monitoring by the patient (31);
evaluating the retrieved physiological data (51) against diagnostic criteria for medical concerns comprised of health disorders with detectable symptoms; and
based on findings (52) from the evaluation made, referring the patient (31) for follow up care to a specialist, wherein the act of referral is performed by the server by electronically transmitting .

15. A method according to Claim 14, further comprising:
interfacing the prescriptive authority to the patient's customary care provider.
